Europäisches Patentamt

European Patent Office

Office européen des brevets

(11) Publication number: **0 134 614**
A1

## (12) EUROPEAN PATENT APPLICATION

(21) Application number: 84201185.0

(22) Date of filing: 15.08.84

(51) Int. Cl.⁴: **F 04 B 43/14**
F 04 B 43/00, A 61 M 5/14
G 01 F 11/08, H 01 L 41/08

(30) Priority: 15.08.83 NL 8302860

(43) Date of publication of application:
20.03.85 Bulletin 85/12

(84) Designated Contracting States:
DE FR GB IT NL SE

(71) Applicant: VITAFIN N.V.
Postbox 895 Rooi Catootje
Willemstad Curacao Netherlands Antilles(NL)

(72) Inventor: Smits, Johannes Gerardus
Groen van Prinstererlaan 12
NL-7521 AZ Enschede(NL)

(74) Representative: Schumann, Bernard Herman
Johan et al,
OCTROOIBUREAU ARNOLD & SIEDSMA Sweelinckplein
1
NL-2517 GK The Hague(NL)

(54) Piezo-electrical micropump.

(57) The invention relates to a micropump (1) comprising at least two valves (10,12) and at least one displacing member (11), said valves and said displacing member being piezo-electrically controllable.

A prior art pump of that type is based on a known principle, namely the use of an inlet-valve and an outlet-valve to be opened and closed selectively, and a displacing member. The valves as well as the displacing member are in the form of so-called bimorphs or monomorphs positioned on an e.g. metal diaphragm, i.e. piezo-electrically controllable elements.

The invention has amongst other for its purpose to provide the possibility to make a micropump in fully integrated form.

In order to realize the above objectives the invention first of all provides a micropump of the type mentioned in the preamble, according to the invention being characterized in that the valves (10,12) and the displacing member (11) are the same in form, connected in series in a channel (3,8,9) and being functionally controllable for obtaining a peristaltical displacing of the fluid to be pumped through said channel.

FIG.1

EP 0 134 614 A1

0134614

-1-

## Short title: Piezo-electrical micropump.

The invention relates to a micropump comprising at least two valves and at least one displacing member, said valves and said displacing member being piezo-electrically controllable.

Such a micropump is known from the article "An Electronically Controlled Piezo-electric Insulin Pump and Valves" by W.J. Spencer e.a. from the "IEEE Transactions On Sonics and Ultrasonics", vol. SU-25, No. 3, May 1978, pages 153 - 156. This prior art pump is based on a known principle, namely the use of an inlet-valve and an outlet-valve to be opened and closed selectively, and a displacing member. The valves as well as the displacing member are in the form of so called bimorphs or monomorphs positioned on an e.g. metal diaphragm, i.e. piezo-electrically controllable elements of a certain kind. Such elements have the property to take e.g. a concave or convex position by application of an electrical field, from a stable rest position, e.g. a flat position, relative to said rest position.

The above-mentioned pump according to Spencer has the disadvantage of providing a pressure-depending dosing.

0134614

Besides special precautions has to be taken in order to make sure that the valves present in the fluid are electrically insulated.

In general the invention has as its object to solve the disadvantages of the prior art.

The invention has for its purpose to provide a micropump of a type such that it can be manufactured by using techniques suitable for mass production.

Furthermore the invention has as its purpose to provide a micropump of a type that can be manufactured in an easy way, so that it is cheap.

It is a further purpose of the invention to provide a micropump of a type offering an exact dosing with an improved pressure-independance.

It is an other purpose of the invention to provide a micropump of a type that is well suited for miniaturization. This may be particularly important for pumps to be implanted, e.g. for the dosed dispensing of insulin to diabetics.

Invention has also for its purpose to provide the possibility to make a micropump in fully integrated form. In this connection one can also think of integrating with the pump of a control circuit by using e.g. a well-known silicon-technology.

In order to realize the above objectives the invention first of all provides a micropump of the type mentioned in the preamble, according to the invention being characterized in that the valves and the displacing member are the same in form, connected in series in a channel and being functionally controllable for obtaining a peristaltical displacing of the fluid to be pumped through said channel.

Particularly such a pump can have the special feature that the micropump substantially consists from a bloc having a channel opening out with separated openings at a surface for interruption at the position of each displacing member/valve.

Particularly an alternative embodiment may be used of which the channel consists of separated parts, each opening out in the surface of said bloc with their ends and being connectable through a displacing member/valve with a previous or next-following channel part, respectively.

Preferably use is made of an embodiment in which the pump comprises a chip provided with the channel, said chip consisting of glass or silicon. Such a variant opens the possibility of using one or more per se known IC-technologies. These technologies are very well known and find application on a large scale in the field of semiconductors, which makes them attractive as all possibilities and limitations thereof are known.

In this connection advantageously use may be made of an embodiment, in which the silicon-chip comprises at least one integrated control circuit connected with the valves/displacing members. At the manufacturing of a pump of this last-mentioned type the construction of the pump may take place in one manufacturing step with the manufacturing of the control circuit, as for both the same technique is used.

Advantageously use may be made of an embodiment, of which each inlet-opening has the form of an annular recess connected with the channel and each outlet-opening is positioned substantially concentrically therewith.

Use may be made of bimorphs, as has been mentioned already above in connection with the article from the IEEE Transactions On Sonics and Ultrasonics. Also an embodiment may be used, in which each valve/displacing member has the shape of a round chip, on which a round piezo-electrical chip is positioned in concentrical relation therewith. The carrying chip may e.g. consist of glass or quartz.

In order to combine a large mechanical effectivity with a small amount of material preferably each piezo-electrical chip extends to at most the contour-line connecting the bending points of the carrying chip coupled with the piezo-electrical chip. In the case in which the edge of the round carrying chip is connected with the corresponding surface of the pumping body in such a way that it is not able to bend there, in this connection for a free carrying chip a radius of the contour-line of about 0.6 of the total free radius of the carrying chip is valid. It will be obvious that this factor will generally have an other value for the combination carrying chip/piezo-electrical chip.

In order to ensure a long life-time of the pump and to guarantee that the fluid to be pumped is not polluted by material of the pump, preferably use is made of an alternative embodiment, in which all surfaces contacting with fluid to be pumped are chemically inert. Potential-carrying parts being in contact with fluid to be pumped are preferably electrically insulated from that fluid. Using silicon as basis material the related surfaces may be provided with a nitride or oxide layer.

The invention will now be more fully explained with reference to the drawing of some embodiments. In the drawing show:

Fig. 1 a first embodiment of a pump according to the invention in a perspective view partly broken away;

Fig. 2 a perspective view of an alternative embodiment of the pump according to fig. 1;

Fig. 3 partly a cross section and partly a perspective view of a part of a pump according to the invention;

Fig. 4 a cross section through a valve/displacing member for the explanation of the operation thereof;

Fig. 5 a perspective view partly broken away of a further embodiment;

Fig. 6 partly a cross section and partly a perspective view of a part of a further embodiment; and

Fig. 7 a schematic plan view of a fully integrated pump according to the invention.

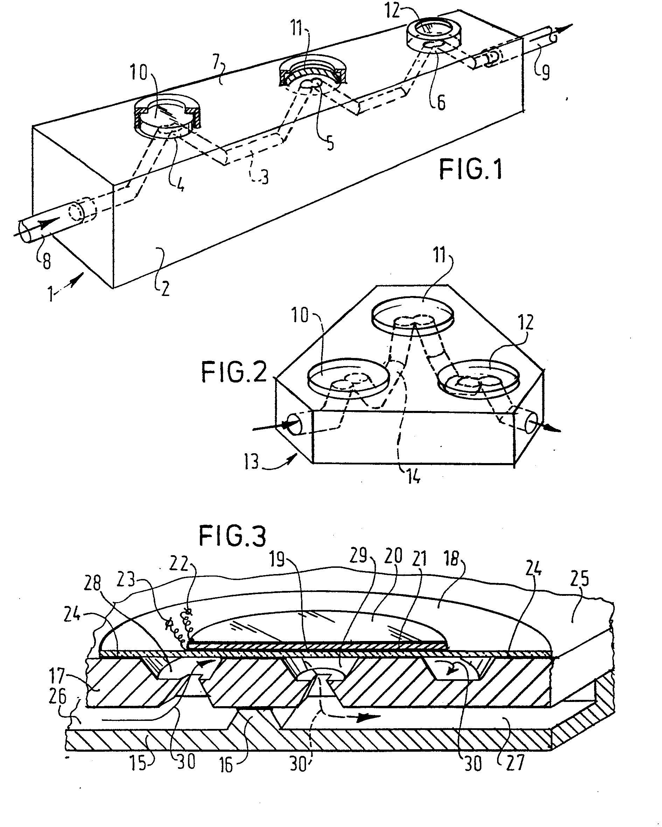

Fig. 1 shows a pump 1. This pump comprises a bloc 2 in which a channel 3 is present opening out at the upper surface 7 at three positions 4, 5, 6. The channel is connected with an inlet conduit 8 and an outlet conduit 9.

At the positions 4, 5, 6 there are piezo-electrical elements 10, 11, 12. These elements are chip- or disc-shaped, they are sealingly coupled with the upper surface 7 and they can be moved by means of not-shown means from a stable rest position, in which they have a flat shape and are positioned on the upper surface 7 to a displaced position in which they exhibit a convex shape.

The embodiment according to fig. 1 operates as follows. First of all the piezo-electrical element 10 is driven electrically, causing to assume a convex shape and to suck fluid to be pumped from the inlet conduit 8 and forming an open connection between that conduit and the channel 3. Then the piezo-electrical element 11 is controlled in a similar manner, causing the fluid to be further displaced from the element 10 to the element 11. Then the element 10 is caused to return into its rest position. At the same time or afterwards the element 12 is brought into its displaced position, in such a way that the fluid to be pumped will be displaced in the direction of that element 12. If subsequently the element 11 is de-energized, the fluid is pumped further into the direction of the outlet conduit 9. Finally the element 12 is brought back in its rest position, due to which the fluid is displaced to the outlet conduit 9, after which the cycle can be repeated.

This very short explanation will suffice to indicate the principle of the peristaltical pump according to the invention. It is noted explicitly that the piezo-electrical elements 10, 11 and 12 are fully identical.

Fig. 2 shows a pump 13 of the same type as the pump 1 according to fig. 1. Pump 13, however, is different of pump 1 in that it is not oblong but generally triangle-shaped. The piezo-electrical elements 10, 11, 12 cooperate with a channel 14 having a shape adapted to the configuration according to fig. 2.

Fig. 3 shows a construction of a part of a pump, of which in each pump, as will be clear, at least three has to be present, consisting from a glass support 15 with local extensions 16 and a silicon-chip 17 carried by said support 15, said chip 17 carrying a glass diaphragm 18 on which a piezo-electrical chip 19 is present, said chip 19 exhibiting an upper electrode 20 and a lower electrode 21 connectable with an electrical control circuit through connections 22, 23. The glass diaphragma is over an annular surface 24 adhered by anodical bonding to the upper surface 25 of the silicon chip 17. Between the glass support 15 and the silicon chip 17 an inlet channel part 26 and an outlet channel part 27 extend. The inlet channel part 26 is connected with an annular recess 28 in the silicon chip 17. An outlet opening 29 is placed in concentrical relation therewith and is connected with the outlet channel part 27. It should be noted that the pump can also be operative in the opposite direction by choice of another type of control. In that case the inlet channel part and the outlet channel part interchange their functions. It will, after the explanation with reference to fig. 1, be clear that the inlet channel part 26 may be connected with an inlet conduit or an outlet channel part of a previous, identical element. Also the outlet channel part may open out at an outlet conduit or an inlet channel part of a next following, identical element.

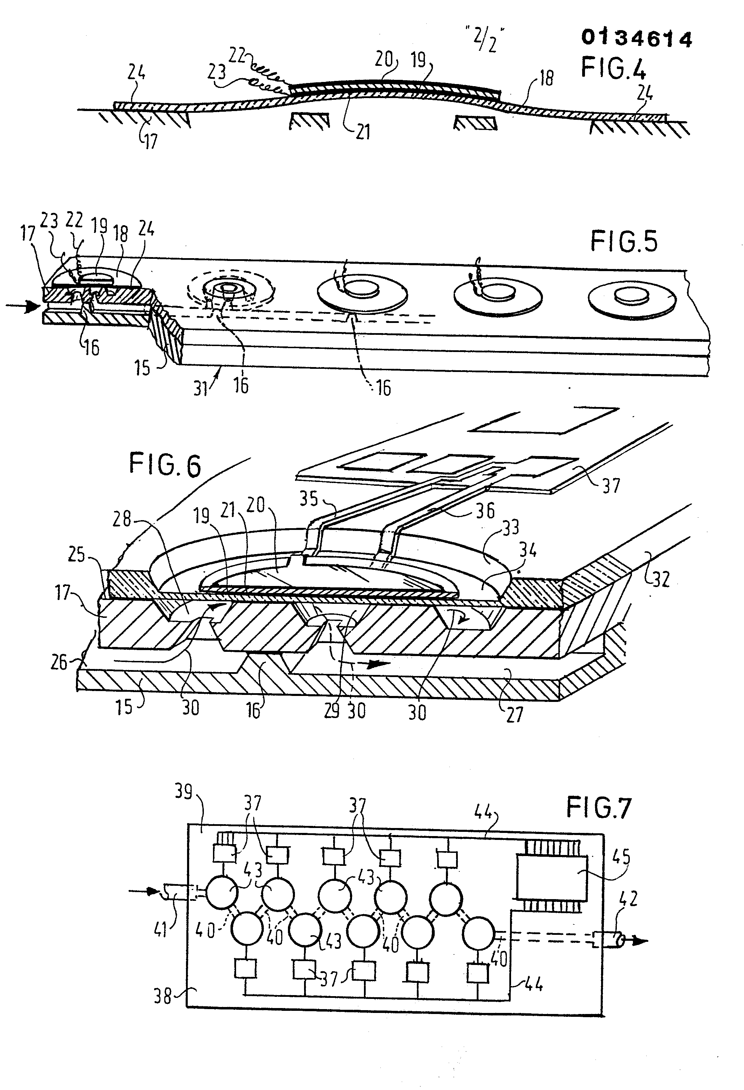

Fig. 4 shows a cross section of the glass diaphragm 18 that over the annular surface 24 is rigidly fixed to the silicon chip 17.

As can clearly be seen in this figure, by applying a voltage between the connections 22 and 23, and thus the application of an electrical field between the electrodes 20 and 21 the piezo-electrical chip can be caused to take a wave-shape together with the glass diaphragm. This wave-shape is caused by the circumstance that due to the applied electrical field the piezo-electrical chip 19 is subjected to an increase of its diameter (application of an opposite electrical field a decrease of its diameter), which, however, is counteracted by the adhesion to the glass diaphragm 18. Due to this wafer-structure the tendency to the increase of diameter can only result in a wave-shape.

In fig. 4 it can further clearly be seen that, in the direction from the annular surface 24 to the centre of the diaphragm 18 firstly the curvature is directed outwardly, and subsequently inwardly. The transition from the outwardly directed curvature to the inwardly directed curvature is a bending point, e.g. in the two-dimensional cross section shape according to fig. 4. It will be clear that the whole diaphragm 18 exhibits a circular bending line.

In order to obtain as large as possible a mechanical effectivity the piezo-electrical chip preferably extends at most to the contour-line connecting the bending points of the combination diaphragm/piezo-electrical chip.

For completeness's sake the attention is drawn to the difference between the flat rest position of the diaphragm 18 according to fig. 3 and the curved, energized position according to fig. 4. After the previous explanation it will be clear that by using a configuration with at least three elements according to fig. 3 a pumping operation can be obtained in correspondence with fig. 1 and 2 and in fig. 3 indicated with arrows 30. As has been indicated before the pumping operation can also take place in the opposite direction by an adapted control.

Fig. 5 shows a pump 31 consisting from a series connection or cascading of elements according to fig. 3.

Fig. 6 shows an integrated variant partly in correspondence with the embodiment according to fig. 3. Corresponding elements have in fig. 6 been indicated with the same reference numerals as in fig. 3.

The silicon chip 17 carries on its upper surface 25 a silicon plate 32. Locally this plate exhibits a recess 33, in such a way that its bottom forms a silicon-diaphragm 34, in exact correspondence with the diaphragm 18 according to fig. 3. The piezo-electrical chip 19 consists from spluttered zinc-oxide and exhibits on its both surfaces the upper electrode 20 and the lower electrode 21, respectively, which last-mentioned electrode in its turn is adhered to the upper surface of the diaphragm 34.

The electrodes 20, 21 are by means of conductive strips 35, 36 connected with an integrated circuit 37 serving as control unit.

Fig. 7 shows a plan view of a fully integrated pump 38 in the form of a laminated chip 39, according to fig. 6; in which a channel 40 having an inlet connection 41 and an outlet connection 42 is present. Pump 38 is provided with ten pumping elements 43 of the type shown in fig. 6. The integrated circuits 37 are by means of bundles conductive strips 44 connected with a common central control circuit 45 of the integrated type.

It should be noted that all services contacting with the fluid to be pumped are chemically inert and electrically insulated. With reference to fig. 6 these are particularly the walls of the inlet channel part 26 and the outlet channel part 27, the annular recess 28, the outlet opening 29 and the lower surface of diaphragm 34. As far as the related surfaces are made of glass, they are already chemically inert. As far as the related surfaces form part of a silicon-element they are oxidized during the manufacturing of the pump, in such a way that a hard silicon-dioxide or quartz layer is present, which is chemically fully inert and electrically insulating.

By a suited, specially adapted control it can be obtained that groups of displacing members/valves are driven, in such a way that "plugs" of fluid are being pumped. It will be clear that thereto a larger number of valves/displacing members are necessary than the basis number of three. It is further remarked that in that case a fault in the form of a leaking valve, and even more leaking valves, will not or at most hardly affect the good pumping operation.

It is further remarked that by a configuration, in which a plurality of pumping units are connected in parallel, but connected to one point with their outputs a mixing of fluids to be pumped may be obtained. By a suitable control even the mixing ratio can be adjusted.

In this connection at last the attention is drawn to the fact that the pumping flow can be chosen by suitably manipulating the following parameters determinitive of the control: the repeating frequency, the fase relation of the several voltages controlling the respective displacing members/valves, the control voltages of the valves/displacing members and at last the number of valves controlled in parallel.

Particularly in case of application as an insulin pump, the pump according to the invention has to be safe, i.e. without electrical driving no fluid is allowed to flow from the reservoir to the outlet. This can be obtained by mounting the diaphragm together with the piezo-disc under such a mechanical bias, that in its rest-position the inner sealing ring (see figs. 3, 4, 6) and the diaphragm are pressed on each other due to the fact that the diaphragm is inclined to assume a concave shape. As an alternative the inner sealing ring can be a little higher, e.g. by means of an additional $SiO_2$-layer in such a way that the diaphragm assumes a convex shape in its rest-position and presses with force on the sealing ring.

-1-

## CLAIMS

1. Micropump comprising at least two valves and at least one displacing member, said valves and said displacing member being piezo-electrically controllable characterized in that the valves and the displacing member are the same in form and connected in a series in a channel and being functionally controllable for obtaining a peristaltical displacing of the fluid to be pumped through said channel.

2. Micropump according to claim 1, characterized in that the micropump substantially consists from a bloc having a channel opening out with separated openings at a surface for interruption at the position of each displacing member/valve.

3. Micropump according to anyone of the preceding claims, characterized in that the channel consists of separated parts, each opening out in the surface of said bloc with their ends and being connectable through a displacing member/valve with a previous or next-following channel part, respectively.

4. Micropump according to anyone of the preceding claims, characterized in that the pump comprises a chip provided with the channel, said chip consisting of glass or silicon.

5. Micropump according to claim 4, <u>characterized in</u> that the silicon-chip comprises at least one integrated control circuit connected with the valves/displacing members.

6. Micropump according to claim 2, <u>characterized in</u> that each inlet-opening has the form of an annular recess connected with the channel and each outlet-opening is positioned substantially concentrically therewith.

7. Micropump according to claim 6, <u>characterized in</u> that each valve/displacing member has the shape of a round chip, on which a round piezo-electrical chip is positioned in concentrical relation therewith.

8. Micropump according to claim 7, <u>characterized in</u> that each piezo-electrical chip extends to at most the contour-line connecting the bending points of the carrying chip coupled with the piezo-electrical chip.

9. Micropump according to anyone of the preceding claims, <u>characterized in</u> that all surfaces contacting with fluid to be pumped are chemically inert.

10. Micropump according to claim 9, <u>characterized in</u> that the related surfaces of elements to be brought under an electrical potential are electrically isolated.

11. Micropump according to claims 9 or 10, <u>characterized in</u> that the related surfaces of elements consisting of silicon are provided with a coating consisting of silicon-dioxide or silicon-nitride.

FIG.1

FIG.2

FIG.3

"2/2"   0134614
FIG.4

FIG.4

FIG.5

FIG.6

FIG.7

## EUROPEAN SEARCH REPORT

European Patent
Office

### DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | FR-A-2 379 055 (COMMISSARIAT A L'ENERGIE ATOMIQUE)<br>* Figures 1,2; page 1, line 17 - page 2, line 19; page 4, line 30 - page 5, line 10; page 6, line 30 - page 7, line 15 * | 1 | F 04 B 43/14<br>F 04 B 43/00<br>A 61 M 5/14<br>G 01 F 11/08<br>H 01 L 41/08 |
| A | | 2,3,9 | |
| Y | DE-A-2 354 249 (AB ORIGINAL-ODHNER)<br>* Figures 1-3; page 1, line 1 - page 2, line 5; page 3, paragraph 6 - page 4, line 3 * | 1 | |
| A | | 7 | |
| A | GB-A-1 555 814 (BATTELLE MEMORIAL INSTITUTE)<br>* Figures 1,3,6,8; page 1, lines 7-17; page 2, line 56 - page 3, line 5; page 3, lines 31-36; page 4, lines 10-16 * | 1-3,6 | **TECHNICAL FIELDS SEARCHED (Int. Cl. ³)**<br><br>F 04 B<br>A 61 M<br>H 01 L<br>G 01 F |
| A | US-A-4 231 287 (PARKER)<br>* Figures 1-9; column 2, line 41 - column 4, line 36 * | 7,8 | |

The present search report has been drawn up for all claims

| Place of search | Date of completion of the search | Examiner |
|---|---|---|
| THE HAGUE | 12-10-1984 | VON ARX H.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82